# EUROPEAN PATENT SPECIFICATION

(11) **EP 2 527 006 B1**
(45) Date of publication and mention of the grant of the patent: **25.10.2017**
(21) Application number: 12168877.4
(22) Date of filing: 22.05.2012
(51) Int. Cl.: A61P 25/18, A61K 9/20, A61K 9/28, A61K 31/554, A61K 47/02

(54) **Quetiapine Formulation Having a Controlled-Release Coating**
Quetiapinformulierung mit Beschichtung zur gesteuerten Freisetzung
Formulation de quétiapine ayant un revêtement à libération contrôlée

(30) Priority: 23.05.2011 TR 201104977
(43) Date of publication of application: 28.11.2012
(73) Proprietor: Sanovel Ilaç Sanayi Ve Ticaret Anonim Sirketi, 34460 Sariyer/Istanbul (TR)
(72) Inventor: Cifter, Ümit, 34460 Istanbul (TR); Türkyilmaz, Ali, 34460 Istanbul (TR); Erdem, Yelda, 34460 Istanbul (TR); Yildirim, Ediz, 34460 Istanbul (TR); Oner, Levent, 34460 Istanbul (TR)
(74) Representative: Sevinç, Erkan

(56) References cited:
- EP-A1- 1 958 617
- WO-A2-2010/001413
- WO-A2-2010/082220
- CHENG A ET AL: "Quetiapine fumarate sustained controlled-released agents combination for treating schizophrenia, contains preset amounts of quetiapine fumarate, organic acid, water soluble polymer, enteric material, wax and water-fast polymer", WPI / THOMSON,, 3 July 2007 (2007-07-03), XP002569065,

## Description

### Field of Invention

The present invention relates to a novel pharmaceutical formulation comprising quetiapine or a pharmaceutically acceptable salt, solvate or hydrate of quetiapine. The present invention more particularly relates to a formulation having a coating, which ensures formulation stability and a controlled release of quetiapine.

### Background of Invention

Quetiapine fumarate, a dibenzothiazepine derivative, is an atypical antipsychotic. It ameliorates the negative and positive symptoms of schizophrenia, without giving rise to extrapiramidal side effects. Similar to clozapine, quetiapine shows moderate dopamine D2-receptor antagonist and potent 5-HT2-receptor antagonist effects. The chemical designation of quetiapine fumarate is 2-[2-(4-dibenzo[b,f][1,4]thiazepine-11-yl-1-piperazinyl)ethoxy] ethanol fumarate, with the following chemical structure of Formula I.

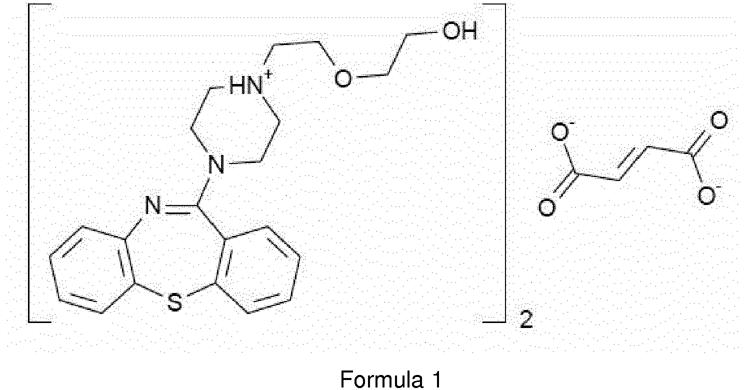

Extended release tablets of quetiapine is marketed under the name Seroquel XR® and is administered orally once a day. These tablets comprise 50 mg, 150 mg, 200 mg, 300 mg or 400 mg quetiapine fumarate as an active agent.

The quetiapine molecule was disclosed in the applications EP0240228 and US4879288.

The patent WO2010001413 discloses a formulation comprising quetiapine and a non-gelling controlled-release polymer, as well as one or more excipients. Said formulation further comprises a controlled-release coating.

The patent EP1958617 discloses a granular formulation used in preparing a formulation. Said formulation comprises a core, containing quetiapine and a binder, as well as a coating layer containing a lubricant.

It is a desired feature to obtain an active pharmaceutical agent in a controlled-release form in the treatment of many diseases. The term 'controlled release' may be defined as reaching desired plasma levels of an active agent of interest throughout a determined period of time, and providing the drug release at a uniform and constant rate. Thus, the drug administration frequency can be lowered.

There are various difficulties associated with developing a controlled-release formulation. One of the problems encountered is dose dumping, as a result of which a drug obtained is released too rapidly. Achieving targeted solubility profiles is also difficult, i.e. it is difficult to control the release rate. For this reason, there may occur variations in the plasma concentrations of active agents, which may lead to toxicity. In addition, there are also daily alterations possible for the active agent in the plasma.

There are various formulations available, which have been developed with quetiapine fumarate. There are various problems, however, associated with quetiapine fumarate formulations.

One of the problems associated with the controlled-release systems is the difficulty in achieving proper release profiles. Particularly the dose dumping may lead to undesired outcomes in terms of the patients. Dose dumping is one of the most significant drawbacks of extended-release dosage forms. The most significant criterion of dose dumping under in vitro conditions is the amount of the active agent released at an earlier time point. Any excessive release of the active agent prior to a determined time interval may lead to uncontrollable damages in terms of a patient.

Producing controlled-release tablets of quetiapine fumarate and similar active agents having low solubility rates, and providing desired release profiles in these tablets are quite difficult. Generating release amounts at desired intervals bears vital importance with respect to patients. Obtaining a desired release profile depends on the convenience of excipients to be selected.

Quetiapine fumarate is also a low-density substance. Therefore the tablet production process of quetiapine fumarate is quite difficult. Tablets produced are prone to erosion and disintegration.

Quetiapine fumarate is to be stored below temperatures of 30°C under normal conditions. Stability-related problems are encountered in temperatures exceeding this temperature point. Failing to prepare a formulation with convenient excipients leads to stability problems.

Accordingly, there is a need towards controlled-release formulations of quetiapine or a pharmaceutically-acceptable salt thereof, which would overcome the problems referred to hereinabove, and would provide drug plasma concentrations equivalent or better than those of the currently-used immediate-release tablet formulations. The formulation according to this invention provides a novel controlled-release formulation, not disclosed in the relevant art yet.

The controlled-release formulation according to this invention both provides plasma levels comparable to the immediate-release form thereof, and gives an advantageous feature in terms of administering the active drug less frequently, e.g. once or twice a day.

Whilst single-layer coatings are applied to already-finished formulations, this does not suffice in providing protection against humidity and therefore blisters, containing aluminum to avoid humidity, are used as well. Blisters, however, are of high costs and thus are not economic.

Carrying out the film coating operation at desired levels of accuracy is very crucial with respect to ensuring the stability of the quetiapine molecule. A preferred coating must both provide protection against environmental effects to ensure stability, and not cause any problems encountered in film coatings, such as wrinkling surfaces, blister and bubble formation, efflorescence, peeling, etc. In this respect, a proper selection and the compatibility of the coating excipient and the plasticizer are crucial.

Due to the reasons specified above, there is a need towards a stable pharmaceutical formulation of quetiapine or pharmaceutically acceptable salts, solvates, or hydrates thereof, providing a proper content uniformity and a desired release profile.

### Object and Brief Description of Invention

The present invention provides a controlled-release formulation of quetiapine, eliminating all aforesaid problems and bringing additional advantages to the relevant prior art.

Accordingly, the main object of the present invention is to obtain a controlled-release formulation comprising quetiapine, which is stable and provides a desired release profile.

Another object of the present invention is to embody a coating, which serves to obtain a desired release profile.

A further object of the present invention is to prevent any dose dumping from occurring in the pharmaceutical formulation by means of the coating embodied according to the present invention.

Yet a further object of the present invention is to obtain a controlled-release formulation with a desired dissolution rate by means of proper coating excipients employed.

A controlled-release pharmaceutical formulation, which is composed of a core and a coating layer, and comprises quetiapine or a pharmaceutically acceptable salt, solvate, or polymorph thereof has been developed to carry out all objects, referred to above and to emerge from the following detailed description.

In a preferred embodiment of the present invention, said novelty is embodied in that
I) the total core weight comprises
   a) sodium citrate dihydrate at 1 to 10% by weight, and
II) the total coating weight comprises
   b) ethyl cellulose at 20 to 45% by weight,
   c) triethyl citrate at 8 to 18% by weight, and
   d) sodium phosphate at 5 to 15% by weight.

According to a preferred embodiment of the present invention, the proportion by weight of sodium citrate dihydrate to sodium phosphate is in the range of 0.01 to 3, preferably in the range of 0.02 to 2.5, and more preferably in the range of 0.04 to 2.3.

According to a preferred embodiment of the present invention, the proportion by weight of ethyl cellulose to triethyl citrate in the coating layer is in the range of 1 to 7, preferably in the range of 1.1 to 6.5, and more preferably in the range of 1.2 to 6.

According to a preferred embodiment of the present invention, the proportion by weight of ethyl cellulose in the coating to the total coating weight is in the range of 10 to 65%, preferably in the range of 15 to 50%, and more preferably in the range of 20 to 40%.

According to another preferred embodiment of the present invention, said quetiapine is in the form of a fumarate salt thereof.

According to another preferred embodiment of the present invention, the excipients used comprise at least one or a mixture of fillers, buffering agents, glidants, and lubricants.

According to a preferred embodiment of the present invention, the filler is at least one of microcrystalline cellulose and lactose monohydrate, or a properly-proportioned mixture thereof.

According to a preferred embodiment of the present invention, the buffering agent is sodium citrate dihydrate. With using sodium citrate dihydrate in the formulation, the desired release profile can be obtained independent from the pH of the medium.

According to another preferred embodiment of the present invention, the proportion by weight of ethyl cellulose in the coating to sodium citrate dihydrate in the core is in the range of 0.05 to 60, preferably in the range of 1 to 50, and more preferably in the range of 2 to 40.

According to a preferred embodiment of the present invention, the glidant is colloidal silicon dioxide.

According to a preferred embodiment of the present invention, the lubricant is magnesium stearate.

A further preferred embodiment according to the present invention provides a method for preparing a pharmaceutical formulation, this method comprising the steps of
a. mixing quetiapine, filler and the controlled-release agent together,
b. forming wet granulation with an alcohol-water mixture,
c. wet milling and drying,
d. sieving the granules,
e. adding lubricant-glidant into the sieved powder mixture obtained with a granular structure and mixing the latter,
f. compressing into tables, and
g. coating the tablets.

According to another preferred embodiment of to the present invention, said pharmaceutical formulation is consisted of the following ingredients only:
a. total core weight, comprising
   a. quetiapine fumarate at 33 to 70% by weight,
   b. ethyl cellulose at 10 to 65% by weight,
   c. sodium citrate dihydrate at 2 to 30% by weight,
   d. lactose monohydrate at 3.0 to 50% by weight,
   e. microcrystalline cellulose at 4.0 to 60% by weight,
   f. colloidal silicon dioxide at 0.2 to 5.0% by weight,
   g. magnesium stearate at 0.1 to 5.0% by weight;
b. total coating layer weight, comprising
   a. ethyl cellulose at 20 to 45% by weight,
   b. triethyl citrate at 8 to 18% by weight, and
   c. sodium phosphate at 5 to 15% by weight.

### Detailed Description of Invention

### Example

| **Ingredients** | **Amount (%) (w/w)** |
|---|---|
| **core** | |
| quetiapine fumarate | 51.2 |
| ethyl cellulose | 22.0 |
| sodium citrate dihydrate | 3.0 |
| lactose monohydrate | 4.6 |
| microcrystalline cellulose | 16.2 |
| colloidal silicone dioxide | 0.6 |
| magnesium stearate | 2.4 |

| **coating** | |
|---|---|
| ethyl cellulose | 22.23 |
| triethyl citrate | 13.41 |
| sodium phosphate | 9.94 |

Ethyl celluloses, commercially available under the trademark Aqualon®, used in the present invention have the following ethoxyl contents:

| **Type** | **Ethoxyl content (%)** |
|---|---|
| N type | 48 - 49.5 |
| T type | 49.6-51.5 |

(see the 2002-document, specifying physical and chemical specifications of the product Aqualon® of the company Herkules, p. 3.)

Ethyl celluloses of type N and T, as indicated in the table, have different ethoxyl contents and show characteristic features depending on these content rates. These two types (N and T) of ethyl cellulose do not jellify. With the invention made, at least one or a properly-proportioned mixture of the type N and T ethyl celluloses are used.

The N type and/or T type ethyl cellulose used in the formulation does not dissolve in water and does not jellify. Specifically-selected excipients are used together with the N type and/or T type ethyl cellulose in the formulation. The hydrophobic sodium citrate dihydrate, among these excipients, generates a proper pH medium around the ethyl cellulose matrix and facilitates its solubility. Thanks to this feature, the release profile of the formulation is brought to the desired level independently from the pH of the medium. On the other hand, sodium phosphate is used as pore-generator and is another excipient which serves to achieve the desired release profile. The dose dumping effect possibly to occur at the initial time points up to two hours of the release profile is controlled by means of the N type ethyl cellulose preferably used in the coating, and the desired release profile is achieved by using a pore generating agent. In fact, it is quite difficult to make a coating with ethyl cellulose. This difficulty, however, is overcome by using triethyl citrate, which is preferred specifically, and by keeping the proportion by weight of triethyl citrate to the weight of the coating in a range of 8 to 18%. Thus, a formulation is obtained, providing desired stability values and a desired release profile.

Thanks to the present invention developed, a stable controlled-release formulation of quetiapine is surprisingly obtained, by which quetiapine is uniformly dispensed in the formulation, a desired release profile is achieved, and the aforesaid problems are solved. Thanks to the formulation developed, the risk of dose dumping is eliminated and a controlled-release formulation is obtained by using ethyl cellulose, which does not jellify. In result, an ideal release profile and stability are obtained by means of properly selected excipients in the core and coating of the controlled-release formulation according to the present invention, which has the desired features.

The formulation is made as follows. Quetiapine, filling agent, and the controlled-release agent are mixed together. Then this mixture is subjected to a wet milling process, together with an alcohol-water mixture. Then, the granules obtained by wet milling and then dried are sieved. Thereafter, lubricant-glidant are added to the sieved powder mixture with a granular form and the resulting mixture is mixed again. Finally, the mixture obtained is compressed into tablets and the tablets obtained are coated.

The coating process is conducted as follows. Sodium phosphate and triethyl citrate are dissolved in water. A homogenous solution is prepared with ethyl cellulose, sodium lauryl sulphate, cetyl alcohol. Both solutions are mixed. The tablets are coated with this solution obtained.

| Time (Minutes) | SEROQUEL PROLONG 200 mg RETARD TABLET | QUETIAPINE SANOVEL XR 200 mg PROLONG RELEASE TABLETS |
|---|---|---|
| | Dissolved (%) | Dissolved (%) |
| 0 | **0,0** | **0,0** |
| 30 | **8,6** | **10,2** |
| 60 | **15,6** | **20,4** |
| 120 | **26,8** | **36,3** |
| 180 | **37,4** | **49,7** |
| 240 | **41,6** | **49,7** |
| 360 | **47,5** | **57,0** |
| 480 | **57,5** | **68,4** |
| 600 | **68,7** | **77,1** |
| 720 | **79,3** | **86,5** |

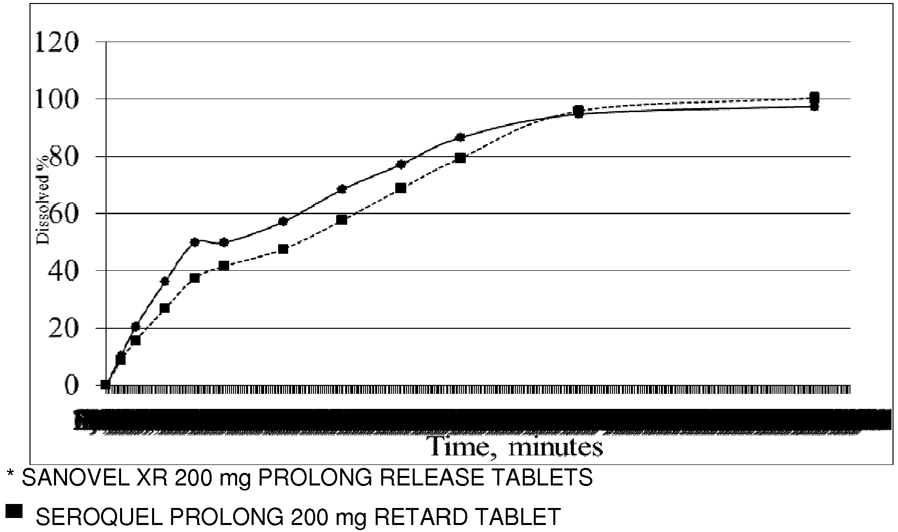

The dissolution tests were performed this condition; Acid stage: 0.1N HCl; 750 ml ;50 rpm, Basket for 2 hours then, The pH 6.8 phosphate buffer phase: 1000 ml, 100 RPM, basket for 12 hours

It is also possible to use the following additional excipients in the formulation.

A binder, e.g. at least one or a mixture of polyvinylpyrrolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives, etc..

A glidant, e.g. at least one or a mixture of colloidal silicone dioxide, talk, aluminum silicate, etc..

A lubricant, e.g. at least one or a mixture of sodium stearyl fumarate, magnesium stearate, polyethylene glycol, stearic acid, metal stearates, boric acid, sodium chloride benzoate and acetate, sodium or magnesium lauryl sulfate, etc..

A colorant, e.g. at least one or a mixture of food, drug, and cosmetic (FD & C) dyes (FD & C blue, FD & C green, FD & C red, FD & C yellow, FD & C lake), ponceau, indigo drug & cosmetic (D & C) blue, indigotine FD & C blue, carmoisine indigotine (indigo Carmine); iron oxides (e.g. iron oxide red, yellow, black), quinoline yellow, flame red, brilliant red (carmine), carmoisine, sunset yellow, etc..

Treatment of diseases, such as the bipolar disease, obsessive-compulsive disorder and schizophrenia, mania, depression, dementia, agitation disorders can be provided with the formulation made according to the present invention.

The protection scope of the present invention is set forth in the annexed claims and cannot be restricted to the illustrative disclosures given above, under the detailed description. Any alternative embodiments to be produced by those skilled in the art according to the basic principles, which are under the protection scope as set forth in the claims, shall be an infringement of the present invention.

## Claims

1. A controlled-release formulation, composed of a core and a coating layer, and comprising quetiapine or a pharmaceutically acceptable salt, solvate, or polymorph of quetiapine, **characterized in that**
I) the total core weight comprises
a) sodium citrate dihydrate at 1 to 10% by weight, and
II) the total coating weight comprises
a) ethyl cellulose at 20 to 45% by weight,
b) triethyl citrate at 8 to 18% by weight, and
c) sodium phosphate at 5 to 15% by weight.

2. The controlled-release formulation according to Claim 1, wherein the proportion by weight of sodium citrate dihydrate to sodium phosphate is in the range of 0.01 to 3, preferably in the range of 0.02 to 2.5, and more preferably in the range of 0.04 to 2.3.

3. The controlled-release formulation according to any of the preceding claims, wherein the proportion by weight of ethyl cellulose to triethyl citrate in the coating layer is in the range of 1 to 7, preferably in the range of 1.1 to 6.5, and more preferably in the range of 1.2 to 6.

4. The controlled-release formulation according to any of the preceding claims, wherein the proportion by weight of ethyl cellulose in the coating layer to the total coating weight is in the range of 10 to 65%, preferably in the range of 15 to 50%, and more preferably in the range of 20 to 40%.

5. The controlled-release formulation according to any of the preceding claims, wherein said quetiapine is in the form of its fumarate salt.

6. The controlled-release formulation according to any of the preceding claims, further containing excipients, comprising at least one or a mixture of fillers, buffering agents, glidants, lubricants.

7. The controlled-release formulation according to claim 6, wherein said filler is at least one of microcrystalline cellulose and lactose monohydrate, or a mixture thereof.

8. The controlled-release formulation according to any of the preceding claims, comprising sodium citrate dihydrate as the buffering agent.

9. The controlled-release formulation according to any of the preceding claims, wherein the weight ratio of ethyl cellulose in the coating layer to sodium citrate dihydrate in the core is in the range of 0.05 to 60, preferably in the range of 1 to 50, and more preferably in the range of 2 to 40.

10. The controlled-release formulation according to any of the preceding claims, comprising colloidal silicone dioxide as the glidant.

11. The controlled-release formulation according to any of the preceding claims, comprising magnesium stearate as the lubricant.

12. A method for preparing a controlled-release formulation according to any of the preceding claims, comprising the steps of
a. mixing quetiapine, filler and the controlled-release agent together,
b. forming wet granulation with an alcohol-water mixture,
c. wet milling and drying,
d. sieving the granules,
e. adding lubricant-glidant into the sieved powder mixture obtained with a granular structure and mixing the latter,
f. compressing into tables, and
g. coating the tablets.

13. The controlled-release formulation according to any of the preceding claims, consisting of the following ingredients only:
a. total core weight, comprising
a. quetiapine fumarate at 33 to 70% by weight,
b. ethyl cellulose at 10 to 65% by weight,
c. sodium citrate dihydrate at 2 to 30% by weight,
d. lactose monohydrate at 3.0 to 50% by weight,
e. microcrystalline cellulose at 4.0 to 60% by weight,
f. colloidal silicon dioxide at 0.2 to 5.0% by weight,
g. magnesium stearate at 0.1 to 5.0% by weight;
b. total coating layer weight, comprising
a. ethyl cellulose at 25 to 45% by weight,
b. triethyl citrate at 8 to 18% by weight, and
c. sodium phosphate at 5 to 15% by weight.

14. The controlled-release formulation according to any of the preceding claims for preventing or treating bipolar disease, obsessive-compulsive disorder and schizophrenia, mania, depression, dementia, agitation disorders in mammalians and particularly in humans.

## Patentansprüche

1. Formulierung zur kontrollierten Freisetzung, bestehend aus einem Kern und einer Hüllschicht, und umfassend Quetiapin oder ein pharmazeutisch akzeptables Salz, Solvat oder Polymorph von Quetiapin, **dadurch gekennzeichnet, dass**
I) das Gesamtkerngewicht folgendes umfasst:
a) 1 bis 10 Gew.-% Natriumcitratdihydrat, und
II) das Gesamtbeschichtungsgewicht folgendes umfasst:
a) 20 bis 45 Gew.-% Ethylcellulose,
b) 8 bis 18 Gew.-% Triethylcitrat und
c) 5 bis 15 Gew.-% Natriumphosphat.

2. Formulierung zur kontrollierten Freisetzung nach Anspruch 1, wobei das Gewichtsverhältnis von Natriumcitratdihydrat zu Natriumphosphat im Bereich von 0,01 bis 3, vorzugsweise im Bereich von 0,02 bis 2,5 und noch bevorzugter im Bereich von 0,04 bis 2,3 liegt.

3. Formulierung zur kontrollierten Freisetzung nach einem der vorhergehenden Ansprüche, wobei das Gewichtsverhältnis von Ethylcellulose zu Triethylcitrat in der Hüllschicht im Bereich von 1 bis 7, vorzugsweise im Bereich von 1,1 bis 6,5 und noch bevorzugter im Bereich von 1,2 bis 6 liegt.

4. Formulierung zur kontrollierten Freisetzung nach einem der vorhergehenden Ansprüche, wobei das Gewichtsverhältnis von Ethylcellulose in der Hüllschicht zum Gesamtbeschichtungsgewicht im Bereich von 10 bis 65 %, vorzugsweise im Bereich von 15 bis 50 % und noch bevorzugter im Bereich von 20 bis 40 % liegt.

5. Formulierung zur kontrollierten Freisetzung nach einem der vorhergehenden Ansprüche, wobei das Quetiapin in Form seines Fumaratsalzes vorliegt.

6. Formulierung zur kontrollierten Freisetzung nach einem der vorhergehenden Ansprüche, ferner enthaltend Exzipienten, umfassend wenigstens eines oder eine Mischung von folgendem: Füller, Puffer, Gleitmittel, Schmiermittel.

7. Formulierung zur kontrollierten Freisetzung nach Anspruch 6, wobei der Füller wenigstens eines von Folgendem: mikrokristalline Cellulose und Lactosemonohydrat oder eine Mischung davon ist.

8. Formulierung zur kontrollierten Freisetzung nach einem der vorhergehenden Ansprüche, umfassend Natriumcitratdihydrat als Puffer.

9. Formulierung zur kontrollierten Freisetzung nach einem der vorhergehenden Ansprüche, wobei das Gewichtsverhältnis von Ethylcellulose in der Hüllschicht zu Natriumcitratdihydrat im Kern im Bereich von 0,05 bis 60, vorzugsweise im Bereich von 1 bis 50 und noch bevorzugter im Bereich von 2 bis 40 liegt.

10. Formulierung zur kontrollierten Freisetzung nach einem der vorhergehenden Ansprüche, umfassend kolloidales Siliciumdioxid als Gleitmittel.

11. Formulierung zur kontrollierten Freisetzung nach einem der vorhergehenden Ansprüche, umfassend Magnesiumstearat als Schmiermittel.

12. Verfahren zur Herstellung einer Formulierung zur kontrollierten Freisetzung nach einem der vorhergehenden Ansprüche, umfassend die Schritte:
a. Vermischen von Quetiapin, Füller und dem Agens zur kontrollierten Freisetzung,
b. Durchführen einer Feuchtgranulation mit einem Alkohol-Wasser-Gemisch,
c. Nassmahlen und Trocknen,
d. Sieben der Körnchen,
e. Zugeben von Schmiermittel/Gleitmittel zu dem gesiebten Pulvergemisch, das mit einer granulären Struktur erhalten wurde, und Vermischen des letzteren,
f. Verpressen zu Tabletten und
g. Beschichten der Tabletten.

13. Formulierung zur kontrollierten Freisetzung nach einem der vorhergehenden Ansprüche, ausschließlich bestehend aus folgenden Bestandteilen:
a) Gesamtkerngewicht, umfassend:
a. 33 bis 70 Gew.-% Quetiapinfumarat,
b. 10 bis 65 Gew.-% Ethylcellulose,
c. 2 bis 30 Gew.-% Natriumcitratdihydrat,
d. 3,0 bis 50 Gew.-% Lactosemonohydrat,
e. 4,0 bis 60 Gew.-% mikrokristalline Cellulose,
f. 0,2 bis 5,0 Gew.-% kolloidales Siliciumdioxid,
g. 0,1 bis 5,0 Gew.-% Magnesiumstearat,
b) Gesamthüllschichtgewicht, umfassend
a. 25 bis 45 Gew.-% Ethylcellulose,
b. 8 bis 18 Gew.-% Triethylcitrat und
c. 5 bis 15 Gew.-% Natriumphosphat.

14. Formulierung zur kontrollierten Freisetzung nach einem der vorhergehenden Ansprüche zur Prävention oder Behandlung von bipolaren Störungen, Zwangsstörungen und Schizophrenie, Manie, Depressionen, Demenz und Agitation bei Säugetieren, insbesondere Menschen.

## Revendications

1. Formulation à libération contrôlée, composée d'un coeur et d'une couche d'enrobage, et comprenant de la quétiapine ou un sel, solvate ou polymorphe pharmaceutiquement acceptable de quétiapine, **caractérisée par le fait que** :
I) le poids total du coeur comprend
a) du citrate de sodium dihydraté à raison de 1 à 10 % en poids, et
II) le poids total de l'enrobage comprend :
a) de l'éthyl cellulose à raison de 20 à 45 % en poids,
b) du citrate de triéthyle à raison de 8 à 18 % en poids, et
c) du phosphate de sodium à raison de 5 à 15 % en poids.

2. Formulation à libération contrôlée selon la revendication 1, dans laquelle la proportion en poids du citrate de sodium dihydraté par rapport au phosphate de sodium se situe dans la plage de 0,01 à 3, de préférence dans la plage de 0,02 à 2,5, et de façon davantage préférée dans la plage de 0,04 à 2,3.

3. Formulation à libération contrôlée selon l'une quelconque des revendications précédentes, dans laquelle la proportion en poids de l'éthyl cellulose au citrate de triéthyle dans la couche d'enrobage se situe dans la plage de 1 à 7, de préférence dans la plage de 1,1 à 6,5, et de façon davantage préférée dans la plage de 1,2 à 6.

4. Formulation à libération contrôlée selon l'une quelconque des revendications précédentes, dans laquelle la proportion en poids de l'éthyl cellulose dans la couche d'enrobage au poids total d'enrobage se situe dans la plage de 10 à 65 %, de préférence dans la plage de 15 à 50 %, et de façon davantage préférée dans la plage de 20 à 40 %.

5. Formulation à libération contrôlée selon l'une quelconque des revendications précédentes, dans laquelle ladite quétiapine se présente dans la forme de son sel fumarate.

6. Formulation à libération contrôlée selon l'une quelconque des revendications précédentes, contenant en outre des excipients, comprenant au moins l'un ou un mélange de charges, d'agents tampons, d'agents de glissement, de lubrifiants.

7. Formulation à libération contrôlée selon la revendication 6, dans laquelle ladite charge est au moins l'un parmi la cellulose microcristalline et le lactose monohydraté, ou un mélange de ceux-ci.

8. Formulation à libération contrôlée selon l'une quelconque des revendications précédentes, comprenant du citrate de sodium dihydraté comme agent tampon.

9. Formulation à libération contrôlée selon l'une quelconque des revendications précédentes, dans laquelle le rapport en poids de l'éthyl cellulose dans la couche d'enrobage au citrate de sodium dihydraté dans le coeur se situe dans la plage de 0,05 à 60, de préférence dans la plage de 1 à 50, et de façon davantage préférée dans la plage de 2 à 40.

10. Formulation à libération contrôlée selon l'une quelconque des revendications précédentes, comprenant du dioxyde de silicone colloïdal comme agent de glissement.

11. Formulation à libération contrôlée selon l'une quelconque des revendications précédentes, comprenant du stéarate de magnésium comme lubrifiant.

12. Procédé de préparation d'une formulation à libération contrôlée selon l'une quelconque des revendications précédentes, comprenant les étapes de
a. mélanger ensemble la quétiapine, la charge et l'agent de libération contrôlée ;
b. former une granulation humide avec un mélange alcool-eau ;
c. broyer par voie humide et sécher ;
d. tamiser les granules ;
e. ajouter un lubrifiant-agent de glissement dans le mélange pulvérulent tamisé obtenu avec une structure granulaire et mélangre ce dernier ;
f. comprimer pour obtenir des comprimés ; et
g. enrober les comprimés.

13. Formulation à libération contrôlée selon l'une quelconque des revendications précédentes, consistant en les ingrédients suivants seulement :
a. poids total du coeur, comprenant :
a. fumarate de quétiapine à raison de 33 à 70 % en poids ;
b. éthyl cellulose à raison de 10 à 65 % en poids ;
c. citrate de sodium dihydraté à raison de 2 à 30 % en poids ;
d. lactose monohydraté à raison de 3,0 à 50 % en poids ;
e. cellulose microcristalline à raison de 4,0 à 60 % en poids ;
f. dioxyde de silicium colloïdal à raison de 0,2 à 5,0 % en poids ;
g. stéarate de magnésium à raison de 0,1 à 5,0 % en poids ;
b. poids total de la couche d'enrobage, comprenant :
a. éthyl cellulose à raison de 25 à 45 % en poids ;
b. citrate de triéthyle à raison de 8 à 18 % en poids ; et
c. phosphate de sodium à raison de 5 à 15 % en poids.

14. Formulation à libération contrôlée selon l'une quelconque des revendications précédentes pour prévenir ou traiter la maladie bipolaire, le trouble obsessionnel compulsif et la schizophrénie, la manie, la dépression, la démence, les troubles d'agitation chez les mammifères et en particulier chez les êtres humains.
